# EUROPÄISCHE PATENTANMELDUNG

(11) **EP 1 010 404 A2**
(43) Veröffentlichungstag der Anmeldung: **21.06.2000**
(21) Anmeldenummer: 99124743.8
(22) Anmeldetag: 13.12.1999
(51) Int. Cl.: A61C 19/00

(54) **Intelligente medizinische Behandlungsvorrichtung**

(30) Priorität: 14.12.1998 DE 19857613
(71) Anmelder: Ellerbrock, Christof, 55218 Ingelheim (DE)
(72) Erfinder: Ellerbrock, Christof, 55218 Ingelheim (DE)
(74) Vertreter: Walz, Erich, Dipl.-Ing.

(57) **Zusammenfassung**

Die Erfindung betrifft eine medizinische Behandlungsvorrichtung (1) zur Durchführung von medizinischen Untersuchungen und Behandlungen. Eine derartige Behandlungsvorrichtung (1) weist eine Vielzahl von Einzelkomponenten (3, 9, 12, 44, 45, 46, 47, 48), bei einer zahnmedizinischen Behandlungsvorrichtung (1) beispielsweise Bohrer (46..48), Fußschalter (3), Sitzverstellung etc. auf. Zur Erhöhung des Leistungs- und Funktionsspektrums einer derartigen Behandlungsvorrichtung (1) wird vorgeschlagen, daß die Behandlungsvorrichtung (1) einen Rechner (11) aufweist, der mit mindestens einer Eingabevorrichtung (6, 7) und mindestens einer Anzeigevorrichtung (6, 7, 8) sowie mit den Einzelkomponenten (3, 9, 12, 44, 45, 46, 47, 48) der Behandlungsvorrichtung (1) gekoppelt ist. Als kombinierte Eingabe-/Ausgabevorrichtung (6, 7, 8) ist ein sogenannter Touch-Screen vorteilhaft, mit dem einerseits die jeweilige Benutzeroberfläche visualisiert werden kann und andererseits durch Berühren bestimmter Bereiche des Bildschirms eine Steuerung der Behandlungsvorrichtung (1) und seiner Einzelkomponenten (3, 9, 12, 44, 45, 46, 47, 48) erfolgen kann.

## Beschreibung

Die Erfindung betrifft eine medizinische Behandlungsvorrichtung zur Durchführung von zahnmedizinischen Untersuchungen und/oder Behandlungen.

Eine derartige Behandlungsvorrichtung kommt beispielsweise im Bereich einer Zahnarztpraxis zum Einsatz. Die Behandlungsvorrichtung umfaßt sowohl den Behandlungsstuhl als auch sämtliche zur Zahnbehandlung erforderlichen Einzelkomponenten, wie beispielsweise Bohrer, Absauger, Beleuchtungsvorrichtung.

Der Erfindung liegt die Aufgabe zugrunde, eine medizinische Behandlungsvorrichtung anzugeben, die eine einheitliche Benutzung der verschiedensten Teilkomponenten auf einfache Weise ermöglicht und ohne großen Aufwand erweiterbar bzw. aktualisierbar ist.

Diese Aufgabe wird durch eine medizinische Behandlungsvorrichtung zur Durchführung von zahnmedizinischen Untersuchungen und/oder Behandlungen, insbesondere eine zahnmedizinische Behandlungsvorrichtung mit Einzelkomponenten, insbesondere Behandlungsstuhl, Bohrer, Kamera- und/oder Röntgenvorrichtung, die jeweils über eine Datenverbindung mit einem Rechner gekoppelt sind, wobei auf dem Rechner die den Einzelkomponenten zugeordnete Benutzeroberflächen gespeichert sind, mit mindestens einer Eingabevorrichtung zur Steuerung der Behandlungsvorrichtung und ihrer Einzelkomponenten und mit mindestens einer Anzeigevorrichtung zur Anzeige von zur Steuerung der Behandlungsvorrichtung erforderlichen Benutzeroberflächen und/oder von Patientendaten.

Die medizinische Behandlungsvorrichtung ist modular aus den Einzelkomponenten aufgebaut und kann daher auf einfache Weise für die unterschiedlichsten Anforderungen aus- oder umgerüstet werden. Das zentrale Kernstück der Behandlungsvorrichtung bildet der Rechner, mit dem sämtliche Einzelkomponenten der zahnmedizinischen Behandlungsvorrichtung über einen Datenbus gekoppelt sind. Die Einzelkomponenten bestehen somit aus einer die mechanischen Funktionen realisierenden Hardware-Einheit, beispielsweise Behandlungsstuhl, Bohrer, Röntgeneinrichtung etc, und aus einer die Steuerung der Hardwarekomponenten ermöglichenden Software-Komponente, die eine Bedienung der Einzelkomponenten sicherstellt. Die Steuerung sämtlicher Funktionen der Behandlungsvorrichtung erfolgt durch ebenfalls mit dem Rechner gekoppelten Eingabevorrichtungen. Insgesamt entsteht somit ein intelligentes Behandlungssystem, welches sowohl die Funktionalität einer zahnmedizinischen Behandlungsvorrichtung als auch deren Ergonomie wesentlich erhöht.

Eine einfache, sichere und intuitive Bedienung der Behandlungsvorrichtung wird dadurch ermöglicht, daß die Eingabevorrichtung und die Ausgabevorrichtung als Touch-Screen ausgebildet sind, wobei der Touch-Screen zur Visualisierung einer jeweiligen Benutzeroberfläche und zum Bedienen der Behandlungsvorrichtung durch Berühren bestimmter Bereiche des Bildschirms des Touch-Screen vorgesehen ist. Ein 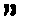 Lernen" der Bedienung beispielsweise neu hinzukommender Einzelkomponenten entfällt.

Eine alternative oder additive Möglichkeit zur umfassenden Bedienung und Nutzung der Behandlungsvorrichtung kann dadurch erzielt werden, daß die Behandlungsvorrichtung als Eingabevorrichtung eine Spracheingabeeinheit zur sprachgesteuerten Bedienung der Behandlungsvorrichtung aufweist, die mit dem Rechner koppelbar ist.

Eine Vernetzung der Behandlungsvorrichtung kann auf einfache Weise dadurch sichergestellt werden, daß der Rechner Mittel zur Kopplung mit weiteren Behandlungsvorrichtungen, mit Rechnern weiterer Behandlungsvorrichtungen und/oder mit einem Verwaltungsrechner zur Verwaltung von Patientendaten aufweist.

Ein modularer Aufbau der Behandlungsvorrichtung, der auch einfach an verschiedenste Erfordernisse angepaßt werden kann, wird dadurch erreicht, daß der Rechner Mittel zur Konfiguration der Behandlungsvorrichtung, zur Aktivierung von Einzelkomponenten und/oder zur Deaktivierung von Einzelkomponenten aufweist.

Die intuitive und sichere Bedienung der Behandlungsvorrichtung wird weiter dadurch gesteigert, daß der Rechner Mittel zur Detektion einer aktuell aktivierten Einzelkomponente der Behandlungsvorrichtung und zur Anzeige der der detektierten Einzelkomponente zugehörigen Benutzeroberftäche aufweist. Hierdurch kann auch eine Mehrfachnutzung vorhandener Bedienvorrichtungen, beispielsweise eine Nutzung eines Fußschalters sowohl zur Steuerung eines Bohrers als auch zum Nachfüllen von Wasser usw. erfolgen.

Ein sofortiger Datentransfer von und zu der Behandlungsvorrichtung beispielsweise zur Übertragung bereits an einem anderen Ort erstellter Röntgenbilder wird dadurch ermöglicht, daß der Rechner der Behandlungsvorrichtung eine Sende-/Empfangskarte, insbesondere eine ISDN-Karte zum Senden und/oder Empfangen von Daten aufweist.

Eine optimale ergonomische Nutzung der möglichen Ein- und Ausgabevorrichtungen wird dadurch sichergestellt, daß die Behandlungsvorrichtung einen Behandlungsstuhl mit einer Kopfstütze aufweist, wobei im Bereich der Kopfstütze eine Anzeigevorrichtung angeordnet ist. Somit hat der Zahnarzt auch bei einer Behandlung eines Patienten zusätzlich die jeweilige Benutzeroberfläche etc. im Blickfeld.

Eine umfassende und schnelle Information des behandelnden Zahnarztes kann in der Weise sichergestellt werden, daß die Behandlungsvorrichtung Mittel zur Darstellung von patientenrelevanten Daten, insbesondere des jeweiligen Behandlungszustandes der Zähne eines Patienten aufweist. Der Zahnarzt kann sich auf diese Wiese beispielsweise einen Überblick über bereits erfolgte Vorbehandlungen sowie die Entwicklung bestimmter Krankheitsprozesse machen.

Die multimedialen Funktionen der Behandlungsvorrichtung werden weiter dadurch vergrößert, daß die Behandlungsvorrichtung ein Spracherkennungssystem aufweist, das insbesondere zum Diktat von durchgeführten Behandlungen dient.

Ein modulares Umrüst- und Erweiterungssystem der Behandlungsvorrichtung entsteht dadurch, daß die Einzelkomponenten der Behandlungsvorrichtung jeweils eine Funktionseinheit aufweisen, die mit dem Rechner koppelbar ist, wobei sämtliche Bedien- und Steuerungsfunktionen der Einzelkomponente über die Eingabevorrichtung(en) der Behandlungsvorrichtung erfolgt.

Im folgenden wird die Erfindung anhand der in den Figuren dargestellten Ausführungsbeispiele näher beschrieben und erläutert.

Es zeigen:
- Fig. 1: ein erstes Ausführungsbeispiel einer zahnmedizinischen Behandlungsvorrichtung als Einzelplatz,
- Fig. 2: ein weiteres Ausführungsbeispiel einer zahnmedizinischen Behandlungsvorrichtung mit vernetztem Rechner,
- Fig. 3: eine schematische Darstellung einer Bildschirmbenutzeroberfläche zur Bedienung und Steuerung der Behandlungsvorrichtung und
- Fig. 4: ein Blockschaltbild eines modular aufgebauten Funktions- und Steuerungssystems für eine zahnmedizinische Behandlungsvorrichtung.

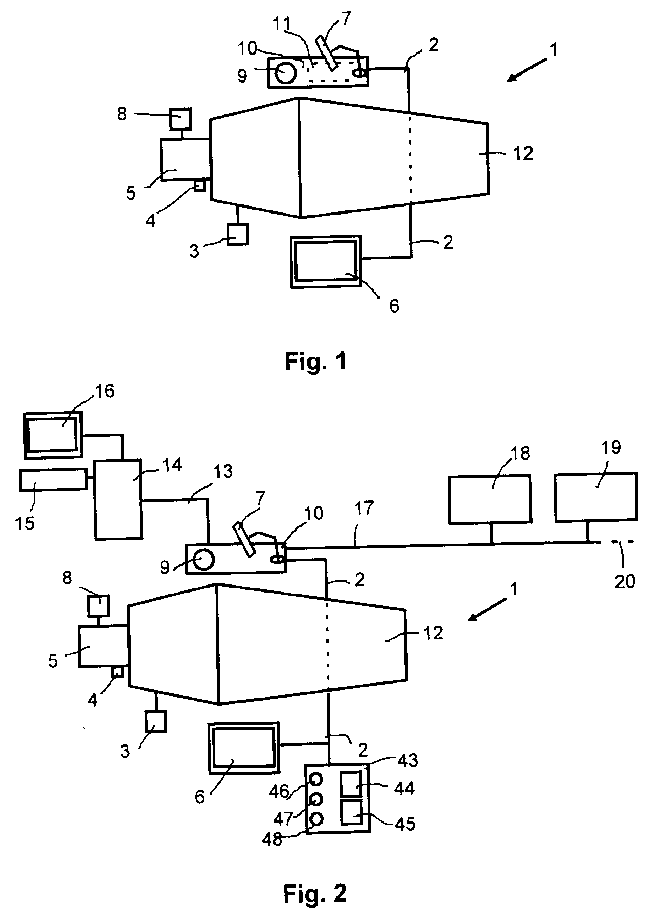

Fig. 1 zeigt eine zahnmedizinische Behandlungsvorrichtung 1 mit einem Behandlunsgstuhl 12 mit einer positionierbaren Kapfstütze 5, mit einer Versorgungseinrichtung 10 und mit an die Versorgungseinrichtung koppelbaren Einzelkomponenten 3, 4, 6, 7, 8, 9. Bei diesen Einzelkomponenten 3, 4, 6, 7, 8, 9 handelt es sich im einzelnen um einen Fußschalter 3, um ein Mikrofon 4, um jeweils als Touch-Screen ausgebildete Bildschirme 6, 7, um ein Display 8 sowie um einen Wasserspender 9. In der Versorgungseinheit 10 ist weiter eine gestrichelt dargestellte Recheneinheit 11 enthalten, mit dem sämtliche Einzelkomponenten 3, 4, 6, 7, 8, 9 gekoppelt sind. So erfolgt bei dem in Fig. 1 dargestellten Ausführungsbeispiel beispielsweise der Anschluß des Touch-Screen 6 über eine Verbindungsleitung 2.

Zentrales Element der Behandlungsvorrichtung 1 ist der integrierte Rechner 11, der netzwerkfähig ist und seitlich, neben dem Behandlungsstuhl 12 in dem Versorungsteil 10 integriert ist. Gesteuert wird die gesamte Behandlungsvorrichtung über den Rechner 11, der sowohl über Touch-Screens 6, 7, als auch über Spracheingabe mit Hilfe des Mikrofons 4. Hierdurch wird ein steriles Arbeiten, bei gleichzeit voller Bedienfähigkeit der Behandlungsvorrichtung sichergestellt. Desweiteren verfügt bzw. steuert der Rechner 11 den medizinischen Datenbus 2, an den alle zusätzlichen Geräte wie z.B. intraorale Kameras oder digitale Röntgensensoren oder Laser angeschlossen werden können. Der Rechner 11 selbst steuert alle Funktionen der kompletten Behandlungsvorrichtung 1, sei es die Sitzposition 12 des Patienten oder die Wasserabgabe in den Becher 9 des Patienten. Über das Betriebssystem ist es möglich neue Geräte direkt in die Einheit einzubinden und ihre Einstellungen via Display oder Sprache festzulegen. Auch 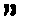 Treiber", d.h. die Daten für Geräte, die keinen direkten Kontakt mit dem Rechner 11 haben, wie Hand- und Winkelstücke bzw. Turbinen, können so in die Einheit eingelesen werden, so daß ihre spezifischen Daten, wie z.B. das Übersetzungsverhältnis vorliegen und so die korrekte Drehzahl des Gerätes auf dem Display 6, 7 innerhalb einer Gerätesysmbolleiste auf den Anzeigeeinheiten 6, 7 ausgegeben wird. Der Rechner 11 erkennt außerdem automatisch, mit welchem Gerät der Zahnarzt gerade arbeitet und hebt dessen Eigenschaften bzw Einstellungen innerhalb der Gerätesysmbolleiste hervor. So ändert sich die Funktion des Fußschalters 3 mit dem aktivierten Gerät. Wählt man aus der Geräteleiste zum Beispiel die Turbine, regelt man mit ihm die Drehzahl, wird die Kamera benutzt, erzeugt man mit ihm ein Standbild oder speichert das Bild ab. Über das integrierte Bussystem wird gleichzeitig während des Betriebs die Funktionsfähigkeit der Einheit und der zusätzlich angeschlossenen Geräte permanent überwacht und Fehlerprotokolle in Dateien auf dem Computer abgelegt. Mit einer im Recher 11 integrierten ISDN-Karte ist es möglich, Daten via ISDN zu versenden bzw. zu empfangen. Auch können Fehlerprotokolle direkt zum technischen Service übertragen werden. An den Behandlungsstuhl 12 wird zusätzlich an der Kopfstütze 5 ein herausklappbares kleines Display 8 angebracht. Hierdurch wird es für den Zahnarzt bei der Benutzung einer intraoralen Kamera ermöglicht, bedingt durch seine Sitzposition, gleichzeitig in den Mund des Patienten und auf das Display zu schauen, um so direkt seine Aufnahmen bewerten zu können. Sowohl auf der Seite der Helferin, als auch auf Zahnarztseite befindet sich ein Display 6 u. 7, das Touch-Screenfähig ist. Mittels dieser Displays 6, 7 läßt sich die gesamte Behandlungsvorrichtung, als auch alle angeschlossenen Geräte steuern.

Fig. 2 ein weiteres Ausführungsbeispiel einer zahnmedizinischen Behandlungsvorrichtung mit einem in der Versorgungseinrichtung 10 integrierten Rechner (vgl. Fig. 1). Die Behandlungsvorrichtung 1 besteht dabei im wesentlichen aus den bereits im Zusammenhang mit Fig. 1 beschriebenen Komponenten. Zusätzlich sind mit dem Rechner der Behandlungsvorrichtung 1 Einheiten 14, 18, 19 vernetzbar. So ist der Rechner der Behandlungsvorrichtung 1 über eine Datenverbindung 13 mit einem zentralen Verwaltungsrechner 14 gekoppelt, der beispielsweise im Sekretariat einer Zahnarztpraxis angeordnet ist und auf dem die Patientendaten der Zahnarztpraxis gespeichert sind. Mit dem Rechner 14 sind eine Tastatur 15 und ein Bildschirm 16 gekoppelt. Weiter sind übert eine Datenverbindung 17 weitere Behandlungsvorrichtungen 18, 19 mit dem Rechner der Behandlungsvorrichtung 1 vernetzt. Über eine gestrichelt eingezeichnete weitere Datenverbindung 20 besteht darüber hinaus die Möglichkeit für eine externe Datenverbindung, beispielsweise eine ISDN-Datenverbindung. Über die Datenverbindung 2 ist darüber hinaus eine Geräteeinheit 43 gekoppelt, die Bohrer 46, 47, eine Zahnpflegeeinheit 48 sowie eine Röntgeneinrichtung 44 und eine Mundkamera 45 aufweist.

Der Rechner 14 stellt insbesondere den Abrechnungscomputer dar, mit dem die Behandlungsvorrichtungen 1, 18,19 über den Datenbus 13 vernetzt sind. Über das Netzwerk 13 können alle an einer Einheit 1, 18, 19 festgelegten Einstellungen an die anderen Einheitem 1, 18, 19 übertragen werden. Die eingesetzte integrierte Software für die Patientendatenverwaltung greift nur noch über das Netzwerk 13 auf die Patientendaten des Verwaltungsrechners 14 zurück. Patientenbezogene Daten wie Kameraaufnahmen und Röntgenbilder werden zahnbezogen, unabhängig vom Abrechnungsrechner gespeichert. Die Sofware ist so ausgelegt, daß dem Zahnarzt patientenrelevante Daten wie Füllungslage, ersetzte Zähne und ähnliches in graphischer Form schnell erkennbar dargestellt werden. Im Zahnschema werden Informationen zu abgelegten Bildern über dem entsprechenden Zahn farblich markiert. Durch Anklicken bzw. Ansprechen des Zahnes öffnet sich ein Dialogfeld über die Bilder die dem Zahn zugeordnet sind und wahlweise über die an diesem Zahn erfolgten Behandlungen, z.B. Tiefe der Wurzelkanäle und ähnliches. Leistungen, die durch den Zahnarzt erbracht werden, werden über das Mikrofon in das 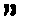 Behandlungsblatt" des Patienten diktiert und dann an die Abrechnungssoftware übergeben. Ein weiteres Ziel der Software ist es auch Heil und Kostenpläne über 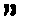 Drag and Drop" Funktionen bzw. Spracheingabe auszufüllen (Zahnangabe, Art der Arbeit), wobei die genaue Berechnung des Planes über den Abrechnungscomputer 14 erfolgt.

Einverständniserklärungen beispielsweise in Form von Unterschriften des Patienten können in der Art erfolgen, daß sie elektronisch erfaßt werden, um den Papierbedarf in der Praxis zu minimieren und das Konzept einer digitalen Praxis konsequent zu verfolgen. Da die zusätzlichen Geräte 44..48, wie z.B. Ultraschall oder Geräte zur Zahnreinigung über den Datenbus 2 angeschlossen werden, entfallen an ihnen alle Einstellelemente wie Schalter und Knöpfe. Alle Einstellmöglichkeiten werden über die Software und das Betriebssystem der Einheit vorgenommen. Hierdurch können diese Zusatzgeräte wie eine 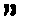 Black-Box" behandelt werden und in ein, in die Einheit 43 integriertes Haltesystem verankert werden. Die Gehäusedimensionen dieser 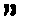 Black-Boxes" sollten der Größe entsprechend aufeinander abgestimmt sein. Ziel einer solche Behandlungseinheit 1 ist es, Systeme von verschiedenen Herstellener optimal nutzen und untereinander kombinieren zum können. So wird es unter anderem für den Zahnarzt möglich sein, sich eine Rehandlungseinheit 1 nach seinen persönlichen Wünschen und Bedürfnissen herstellerunabhängig zusammenzustellen, um so einen für seine Bedürfnisse optimalen Arbeitsplatz zu erhalten, bei dem alle notwendigen Behandlungsgeräte zentral an der Einheit vorhanden sind. Weiterhin erfolgt erstmalig eine Integration von Kommunikationsgeräten in ein Behandlungsgerät, was gerade in Zukunft für die optimale Patientenbetreuung immer bedeutsamer wird.
Mit der Unterstützung der Spracheingabe 4, 11wird dem Zahnarzt ein Instrument in die Hand gelegt, das ihm multiple Vorteile bringt. Zum einen eine Zeiteinsparung, da nicht mehr die erbrachten Leistungen und Notizen von Hand in die Kartei eingetragen werden müssen, sondern während der laufenden Behandlung nebenher einfach diktiert werden können. Hieraus und der Tatsache, daß die diktierten Informationen sofort auf den Displays 6, 7, 8 angezeigt werden, resultiert die Sicherheit, daß die erbrachte Leistung auch erfaßt wurde und so mit der Krankenkasse abgerechnet werden kann und nicht von der Herlferin vergessen wird, die Leistung in den Abrechnungscomputer 14 zu übertragen. Auch ist es so möglich vollkommen steril die Behandlungseinheit 1 und alle angeschlossenen Geräte zu kontrollierten und zu steuern. Weiterhin können mit der Spracheingabe mehrere Befehle gekoppelt sein, so daß das Arbeiten mit dem Computer insgesamt effizienter wird. Die zentrale übersichtliche Steuerung aller Komponenten im Verbund mit deren Darstellung auf dem Display erleichtert dem Behandler die Handhabung der einzelnen Geräte, da kein 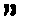 Umdenken" in deren Funktionssteuerung notwendig ist.

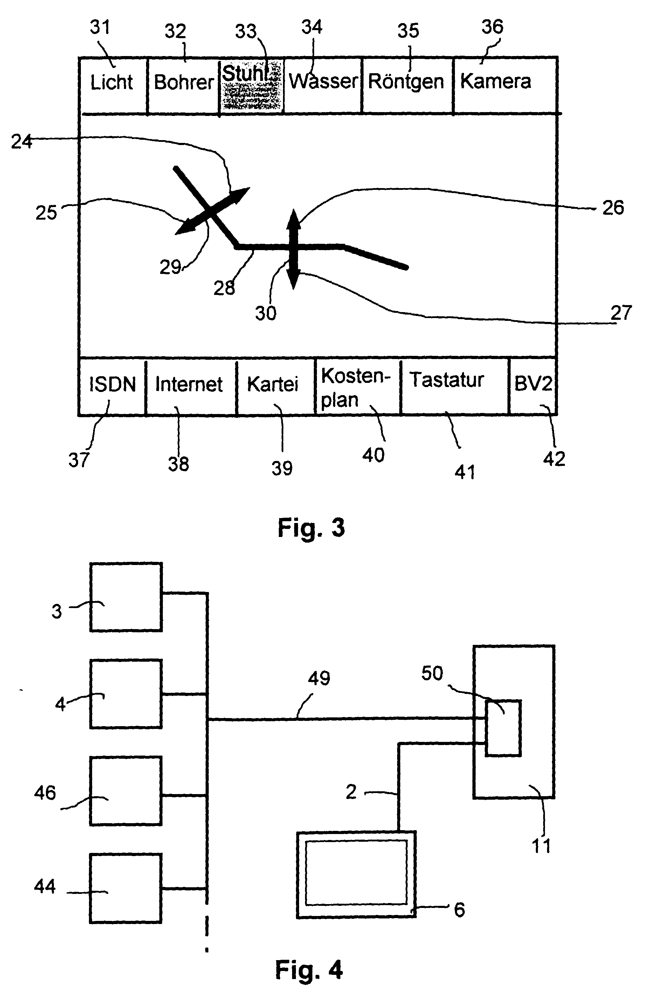

Fig. 3 eine schematische Darstellung einer Bildschirmbenutzeroberfläche zur Bedienung und Steuerung der Behandlungsvorrichtung, wie sie beispielsweise auf den im Zusammenhang mit den Figuren 1 und 2 gezeigten und beschriebenen Touch-Screens verwendet werden. Der Bildschirm ist in drei Bildschirmbereiche unterteilt, wobei im oberen und unteren Bildschirmbereich sich jeweils verschiedene Menüpunkte 31..36 zu verschiedenen Einzelkomponenten der zahnmedizinischen Behandlungsvorrichtung und verschiedene Menüpunkte 37..42 für einen Zugriff auf jeweils vorhandene Medien, z.B. ISDN, Internet, Patientenkartei etc. befinden. Im mittleren Bildschirmbereich der in Fig. 3 dargestellten Benutzeroberfläche ist eine schematischen Darstellung eines Behandlungsstuhles 28 dargestellt, wobei durch Pfeile 29, 30 die die jeweiligen Benutzeroptionen zur Einstellung der Rückenlehne oder Kopfstütze sowie der Sitzhöhe des Behandlungsstuhls dargestellt sind. Eine Verstellung der Sitzposition kann auf einfache Weise dadurch erfolgen, daß der Benutzer, beispielsweise der Zahnarzt auf dem Touch-Screen den Pfeil 29 in den Bereichen 24 berührt, wodurch eine Aufwärtsstellung der Rückenlehne oder der Kopfstütze des Behandlungsstuhls bewirkt wird. Eine flachere Eistellung der Rückenlehne oder der Kopfstütze erfolgt durch Berühren des Pfeils 29 im Bereich der Pfeilspitze 25. Analoges gilt für die Verstellung der Sitzhöhe, wo durch Berühren des Pfeils 30 in Bereichen 26, 27 eine Höher- oder Tieferstellung des Behandlungsstuhles erfolgen kann. Anstelle einer Berührung der Pfeile auf dem Bildschirm kann eine Steuerung der Position des Behandlungsstuhles auch mit Hilfe von Sprachsteuerung über das Mikrofon 4 erfolgen.

Fig. 4 zeigt ein Blockschaltbild eines modular aufgebauten Funktions- und Steuerungssystems für eine zahnmedizinische Behandlungsvorrichtung. Der modulare Charakter der Behandlungsvorrichtung wird dadurch erzielt, daß mit einer Anwendersoftware 50, die auf einem Rechner 11 gespeichert ist, über eine Datenverbindung 49, insbesondere einen Datenbus verschiedene Einzelkomponenten 3, 4, 46, 44 der Behandlungsvorrichtung gekoppelt sind. Bei den Einzelkomponenten handelt es sich beispielsweise um einen Fußschalter 3, um ein Mikrophon 4, um einen Bohrer 46 sowie um eine Röntgenvorrichtung 44. Über eine Verbindungsleitung 2 ist darüber hinaus eine Anzeigevorrichtung 6 mit dem Rechner 11 gekoppelt.

Fig. 4 veranschaulicht nochmals die modular aufgebaute Grundstruktur der zahnmedizinischen Behandlungsvorrichtung. Die Funktionen der jeweiligen Einzelkomponenten 3, 4, 46, 44 werden dabei durch ein Zusammenspiel der jeweiligen Hardwarekomponenten wie Fußschalter 3, Bohrer 46 und Röntgenvorrichtung 44 und durch entsprechende Softwarekomponenten realisiert, die in der Anwendersoftware 50 des Rechners 11 eingebettet sind. Die Softwarekomponenten enthalten die für eine Bedienung der Hardwarekomponenten erforderlichen Benutzeroberflächen. Hierzu sind in der Anwendersoftware den Hardwarekomponenten zugeordnete Datenobjekte enthalten, die jeweils ein Abbild der realen Funktionalität der zugeordneten Hardwareobjekte aufweisen. Eine möglichst ergonomische und intuitive Bedienung und Steuerung der Hardwarekomponenten erfolgt dabei über graphische auf dem Bildschirm 6 dargestellte Benutzeroberflächen.

Zusammenfassend betrifft die Erfindung somit eine zahnmedizinische Behandlungsvorrichtung zur Durchführung von zahnmedizinischen Untersuchungen und Behandlungen. Eine derartige Behandlungsvorrichtung weist eine Vielzahl von Einzelkomponenten, beispielsweise Bohrer, Fußschalter, Sitzverstellung auf. Zur Erhöhung des Leistungs- und Funktionsspektrums einer derartigen Behandlungsvorrichtung wird vorgeschlagen, daß die Behandlungsvorrichtung einen Rechner aufweist, der mit mindestens einer Eingabevorrichtung und mindestens einer Anzeigevorrichtung sowie mit den Einzelkomponenten der Behandlungsvorrichtung gekoppelt ist. Als kombinierte Eingabe-/Ausgabevorrichtung ist ein sogenannter Touch-Screen vorteilhaft, mit dem einerseits die jeweilige Benutzeroberfläche visualisiert werden kann und andererseits durch Berühren bestimmter Bereiche des Bildschirms eine Steuerung der Behandlungsvorrichtung und seiner Einzelkomponenten erfolgen kann.

## Patentansprüche

1. Medizinische Behandlungsvorrichtung (1) zur Durchführung von medizinischen Untersuchungen und/oder Behandlungen mit Einzelkomponenten (3, 9, 12, 44, 45, 46, 47, 48), insbesondere zahnmedizinische Behandlungsvorrichtung (1) mit Behandlungsstuhl (12), Bohrer (46, 47, 48), Kamera- (44) und/oder Röntgenvorrichtung (45), wobei die Einzelkomponenten (3, 9, 12, 44, 45, 46, 47, 48) jeweils über einen Datenbus (2) mit einem Rechner (11) gekoppelt sind, wobei auf dem Rechner (11) die den Einzelkomponenten (3, 9, 12, 44, 45, 46, 47, 48) zugeordnete Benutzeroberflächen gespeichert sind, und mit mindestens einer Eingabevorrichtung (3, 4, 6, 8) zur Steuerung der Behandlungsvorrichtung (1) und ihrer Einzelkomponenten (3, 9, 12, 44, 45, 46, 47, 48) und mit mindestens einer Anzeigevorrichtung (6, 7) zur Anzeige von zur Steuerung der Behandlungsvorrichtung (1) und/oder der Einzelkomponenten (3, 9, 12, 44, 45, 46, 47, 48) erforderlichen Benutzeroberflächen.

2. Behandlungsvorrichtung nach Anspruch 1,
**dadurch gekennzeichnet,**
daß die Eingabevorrichtung und die Ausgabevorrichtung als Touch-Screen (6, 7) ausgebildet sind, wobei der Touch-Screen (6, 7) zur Visualisierung einer jeweiligen Benutzeroberfläche vorgesehen ist, wobei die Benutzeroberfläche graphische Bereiche (24..42) aufweist, die bei Berührung einen zur Erzeugung eines Steuerbefehls an den Rechner (11) zur Ausführung eines dem graphischen Bereich (24..42) zugeordneten Befehls vorgesehen sind.

3. Behandlungsvorrichtung nach einem der Ansprüche 1 oder 2,
**dadurch gekennzeichnet,**
daß die Behandlungsvorrichtung (1) als Eingabevorrichtung eine Spracheingabeeinheit (4) zur sprachgesteuerten Bedienung der Behandlungsvorrichtung (1) aufweist, die mit dem Rechner (11) koppelbar ist.

4. Behandlungsvorrichtung nach einem der Ansprüche 1 bis 3,
**dadurch gekennzeichnet,**
daß der Rechner (11) Mittel zur Kopplung mit weiteren Behandlungsvorrichtungen (18, 19), mit Rechnern weiterer Behandlungsvorrichtungen und/oder mit einem Verwaltungsrechner (14) zur Verwaltung von Patientendaten aufweist.

5. Behandlungsvorrichtung nach einem der Ansprüche 1 bis 4,
**dadurch gekennzeichnet,**
daß der Rechner (11) Mittel zur Konfiguration der Behandlungsvorrichtung (1), zur Aktivierung von Einzelkomponenten (3, 9, 12, 44, 45, 46, 47, 48) und/oder zur Deaktivierung von Einzelkomponenten (3, 9, 12, 44, 45, 46, 47, 48).

6. Behandlungsvorrichtung nach einem der Ansprüche 1 bis 5,
**dadurch gekennzeichnet,**
daß der Rechner (11) Mittel zur Detektion einer aktuell aktivierten Einzelkomponente der Behandlungsvorrichtung (1) und zur Anzeige der der detektierten Einzelkomponente (3, 9, 12, 44, 45, 46, 47, 48) zugehörigen Benutzeroberfläche aufweist.

7. Behandtungsvorrichtung nach einem der Ansprüche 1 bis 6,
**dadurch gekennzeichnet,**
daß der Rechner (11) der Behandlungsvorrichtung eine Sende-/Empfangskarte, insbesondere eine ISDN-Karte zum Senden und/oder Empfangen von Daten aufweist.

8. Behandlungsvorrichtung nach einem der Ansprüche 1 bis 7,
**dadurch gekennzeichnet,**
daß die Behandlungsvorrichtung (1) einen Behandlungsstuhl (12) mit einer Kopfstütze (5) aufweist, wobei im Bereich der Kopfstütze (5) eine Anzeigevorrichtung (8) angeordnet ist.

9. Behandlungsvorrichtung nach einem der Ansprüche 1 bis 8,
**dadurch gekennzeichnet,**
daß die Behandlungsvorrichtung (1) Mittel zur Darstellung von patientenrelevanten Daten, insbesondere des jeweiligen Behandlungszustandes der Zähne eines Patienten aufweist.

10. Behandlungsvorrichtung nach einem der Ansprüche 1 bis 9,
**dadurch gekennzeichnet,**
daß die Behandlungsvorrichtung (1) ein Spracherkennungssystem aufweist, das insbesondere zum Diktat von durchgeführten Behandlungen dient.

11. Behandlungsvorrichtung nach einem der Ansprüche 1 bis 10,
**dadurch gekennzeichnet,**
daß die Einzelkomponenten (3, 9, 12, 44, 45, 46, 47, 48) der Behandlungsvorrichtung (1) jeweils eine Funktionseinheit aufweisen, die mit dem Rechner (11) koppelbar ist, wobei sämtliche Bedien- und Steuerungsfunktionen der Einzelkomponente (3, 9, 12, 44, 45, 46, 47, 48) über die Eingabevorrichtung(en) (3, 4, 6, 8) der Behandlungsvorrichtung (1) erfolgen.
